# EUROPEAN PATENT SPECIFICATION

(11) **EP 4 633 789 B1**
(45) Date of publication and mention of the grant of the patent: **28.10.2026**
(21) Application number: 23833097.1
(22) Date of filing: 15.12.2023
(51) Int. Cl.: B01J 8/02, C07C 31/04, C07C 29/15

(54) **METHANOL SYNTHESIS CONVERTER AND PROCESS**
KONVERTER FÜR DIE METHANOLSYNTHESE UND DESSEN VERFAHREN
CONVERTISSEUR DE SYNTHÈSE DE MÉTHANOL ET SON PROCÉDÉ

(30) Priority: 16.12.2022 EP 22214315
(43) Date of publication of application: 22.10.2025
(73) Proprietor: CASALE SA, 6900 Lugano (CH)
(72) Inventor: MOREO, Pietro, Lugano 6900 (CH); MUSCIONICO, Isabella, 22030 Orsenigo (CO) (IT); POLETTI, Riccardo, 6932 Breganzona (CH)
(74) Representative: M. Zardi & Co S.A.
(86) International application number: PCT/EP2023/086148
(87) International publication number: WO 2024/126824

(56) References cited:
- WO-A1-2019/121155
- JP-A- S63 283 741
- US-A- 4 714 592

## Description

### Field of application

The invention is in the field of methanol synthesis. In particular, the invention relates to a methanol synthesis converter and to a process for the synthesis of methanol.

### Prior art

The industrial preparation of methanol starts in a front-end section of a methanol plant wherein a make-up gas containing carbon oxides CO, CO₂ and hydrogen H₂ is obtained via reforming or partial oxidation of a hydrocarbon feedstock. The hydrocarbon feedstock subjected to reforming can come from different sources but it usually comprises one or more of the following: natural gas, coal, pet-coke, waste and/or biomass.

The conversion of the make-up gas into crude methanol is carried out in one or more methanol converter(s) which is/are part of a methanol synthesis loop. The converters are complex equipment because the reaction that leads to the synthesis of methanol is highly exothermic and the converter must be able to withstand high temperature and high pressure typically from 200 °C to 300 °C and from 50 to 100 bar.

Methanol converters include one or more catalytic bed(s) containing a catalyst and they can be provided with one or more cooling element(s) immersed in the catalytic mass and/or with one or more heat exchanger(s) arranged downstream the catalytic bed(s) to remove the heat developed by the methanol synthesis reaction.

In the art, the catalytic converters provided with cooling elements arranged in the catalytic mass are referred to as isothermal converters in view of the fact that the temperature of the reaction that leads to the synthesis of methanol is kept within a suitable temperature range. On the other hand, converters not provided with cooling elements within the catalytic mass are referred to as adiabatic converters.

Commonly a methanol synthesis loop includes, together with an isothermal converter and/or an adiabatic converter, additional processing units e.g. one or more heat exchangers upstream or downstream of the converter. Said one or more processing units are not part of the catalytic converter.

The performance of a catalytic converter can be evaluated by taking into account an active volume of the converter which in simple terms is the volume of the converter that actively contributes to the synthesis of methanol. The remainder volume can be termed inactive volume. The active volume is the sum of several contributions including the volume occupied by the catalyst bed(s) and the volume occupied by the cooling elements immersed in the catalyst.

In practice, for assessing the performance of the converter, the active volume of the converter is divided by the internal volume of the converter to provide a parameter which is referred to as actively utilized volume ratio. Said ratio is an indirect indication of the proper design of the methanol converter and the higher is the ratio, the lower are CAPEX and the better is for the optimisation of the cost.

Unfortunately, in the adiabatic converters and isothermal converters of the prior art not all the internal volume of catalytic converters is active towards the synthesis of methanol. This is the consequence of mechanical and fluid dynamic constraints and/or maintenance requirements.

In practice, the catalytic converters of the current art are designed with a central cavity which is substantially an empty volume not filled with catalyst and which is inactive towards the synthesis of methanol. It follows that, in view of the current design, the converters are characterised by a low actively utilized volume ratio which results in an unoptimized design.

US 4,714,592 discloses a radial flow reactor including a heat exchange apparatus within the catalytic bed. WO 2019/121155 discloses a multi-bed catalytic converter; JP S63 283741 discloses a contact reactive device for ammonia, methanol or the like.

### Summary of the invention

The invention aims to overcome the above drawbacks of the prior art. Among others, the invention faces the problem of designing a methanol converter having a higher active volume or higher actively utilized volume ratio compared to the converters of the current art.

Accordingly, one aspect of the present invention is a catalytic converter for the synthesis of methanol according to claim 1.

The catalytic converter for the synthesis of methanol comprises a pressure vessel which includes a catalytic bed having an annular configuration and an effluent heat exchanger. Said effluent heat exchanger is installed in an empty central cavity of the converter and is arranged to transfer heat from a hot methanol-containing gas exiting the catalytic bed to a cooling medium.

The converter of the invention includes a catalytic bed. Said catalytic bed being configured as an adiabatic bed or as an isothermal bed

An isothermal catalytic bed is equipped with one or more cooling elements, preferably in the form of heat exchanger plates, arranged within the catalyst bed to remove reaction heat from the catalyst. An adiabatic bed has no cooled heat exchange elements immersed in the catalyst; a heat exchanger is preferably provided downstream an adiabatic catalytic bed to remove heat of reaction from the effluent.

The effluent heat exchanger is a feed-effluent heat exchanger arranged to transfer heat from said hot methanol-containing gas effluent from the catalytic bed to a syngas feed, so that said syngas feed is preheated before entering said catalytic bed.

The volume occupied by the feed effluent heat exchanger within the converter is countable for the definition of the active volume.

A further aspect of the invention is a process for the synthesis of methanol according to the claims.

The advantages of the invention are the following.

The inactive volume of the converter is reduced by the installation in the central cavity of the feed-effluent heat exchanger. It follows that also the actively utilized volume ratio of the converter is increased which is particularly beneficial for the investment cost of the synthesis loop.

A compact, simplified and more practical converter can be obtained particularly in view of the plurality of processing functions which can be integrated into a single pressure vessel. For instance, the converter of the invention can be configured to carry out a series of functions including: cooling, heating and in some embodiments also condensation.

The converter of the invention is particularly suited to be applied in small-size methanol plants and methanol plants powered at least in part by renewable energy sources.

The vessel of the converter can be designed with a slightly bigger diameter than the traditional converters. This marginal increment in the diameter of the converter enables to include of one or more additional processing unit(s) within the volume of the converter. It follows that the costs of external equipment and piping together with the CAPEX are reduced. Further, the pressure losses and the energy consumption (OPEX) are also reduced.

### Description of the invention

In a broad aspect, the invention concerns a catalytic converter for the synthesis of methanol comprising a pressure vessel which includes a catalytic bed having an annular configuration and an effluent heat exchanger.

The effluent heat exchanger is installed in the empty central cavity of the converter and is arranged to transfer heat from a hot methanol-containing gas effluent from the catalytic bed to a cooling medium.

The cooling medium may be unreacted syngas feed to the converter itself (process stream) or an external medium. Suitable external cooling fluids may include: cooling water, demi-water, boiler feed water which can be used in the process for recovery of energy or for steam production.

In a highly preferred embodiment said effluent heat exchanger is a feed-effluent heat exchanger arranged to transfer heat from a hot methanol-containing gas effluent from the catalytic bed to an unreacted syngas feed to the converter, so that said converter feed is preheated before entering the catalytic bed.

Said effluent heat exchanger comprises a first side for the syngas feed and a second side for the hot gas effluent. An output of said first side is in communication with an inlet of said catalytic bed and an inlet of said second side is in communication with an outlet of said catalytic bed.

Said effluent heat exchanger, according to various application, may be one of the following.

In a first option said effluent heat exchanger is a feed-effluent heat exchanger, as above defined, or a unit of the same, in case the feed-effluent heat exchanger is a multi-unit apparatus or is part of a heat exchanger train. In general terms, a feed-effluent heat exchanger is understood as an apparatus including one or more units arranged to cover a given thermal duty, i.e. heat that must be transferred from the hot effluent to the incoming gas. The heat exchanger arranged in the pressure vessel and within the cavity of the annular bed may be, according to various embodiments, the entire feed-effluent heat exchanger, arranged to fully cover the thermal duty, or a unit thereof arranged to cover a percentage of said duty.

In an interesting embodiment, said effluent heat exchanger is a plate type heat exchanger with heat exchange elements in the form of plates. Said heat exchange plates are relatively thin, flat heat exchange bodies having a length and width predominant over the thickness.

In a second option said effluent heat exchanger includes a liquid-gas separator arranged downstream of the feed-effluent heat exchanger.

In a third option said effluent heat exchanger includes a feed-effluent heat exchanger and a further heat exchanger arranged to remove additional heat from the hot reaction effluent with an external cooling medium such as cooling water.

In another option said effluent heat exchanger includes a feed-effluent heat exchanger, an additional heat exchanger and a liquid-gas separator.

The converter of the invention can comprise one or more catalytic bed(s).

Preferably the catalytic converter comprises only a single catalytic bed (single-bed converter). A particularly preferred embodiment is a single-bed isothermal converter having only one catalytic bed which is provided with cooling elements immersed in the catalytic mass to keep the temperature of the reaction zone within a target range. More preferably said cooling elements are in the form of heat exchange plates internally traversed by a cooling medium. The cooling medium is preferably water.

In some embodiments the catalytic converter comprises a single or multiple adiabatic bed(s) wherein no cooling element is provided within the catalytic mass.

In some embodiments the catalytic converter comprises multiple catalytic beds wherein dedicated cooling elements are immersed into each of the catalytic beds so to keep the reaction temperature in the respectively catalytic beds within a target range (isothermal or pseudo-isothermal range).

According to an alternative embodiment the converter is provided with multiple catalytic beds wherein some of the catalytic beds are isothermal beds whilst others are adiabatic beds without cooling elements within the catalytic mass.

According to an embodiment the converter comprises two catalytic beds arranged in series one downstream of the other wherein the first catalytic bed is an isothermal catalytic bed and the second catalytic bed is an adiabatic bed.

According to an embodiment the converter comprises two catalytic beds arranged in series one downstream of the other wherein the first catalytic bed is an adiabatic bed and the second catalytic bed is an isothermal catalytic bed.

According to various embodiments, said feed-effluent heat exchanger is a shell and tube heat exchanger comprising tubes housed within a cylindrical shell or said effluent heat exchanger is a plate type heat exchanger wherein the cooling elements are in the form of plates.

The converter can further comprise a methanol condenser contained in said pressure vessel, wherein said methanol condenser is arranged downstream of and in fluid communication with said feed-effluent heat exchanger, so to receive a cooled methanol-containing gas effluent from said second side of said heat exchanger and to produce a liquid methanol-containing product.

The invention can be applied to the making of a new converter and/or in revamping of pre-existing chemical plants especially when catalytic converters of compact sizes are required and limited space is available for their installation. In some embodiments, a methanol converter according to the invention can replace an existing methanol converter during a revamping procedure.

In a further aspect of the invention, a hydrocarbon or a biomass feedstock is subjected to a reforming step to yield a syngas feed comprising a mixture of carbon oxides and hydrogen and the syngas feed is then subjected to a heating step and subsequently reacted over a catalyst to produce a hot methanol containing gas. In some embodiments, the production of the syngas feed may include gasification of a suitable feedstock, preferably a biomass feedstock. The reforming step may be partially or fully substituted by said gasification step.

The hot methanol containing gas is then cooled and transferred to a condensation step and said heating of the syngas feed and the cooling of said hot methanol containing gas are carried out in an effluent heat exchanger so that the heat retained by the hot methanol containing gas is indirectly transferred to the syngas feed.

The reaction of the syngas feed over the catalyst, the heating of said syngas feed and the cooling of said hot methanol containing gas are carried out in the above-disclosed catalytic converter which can be configured to operate under isothermal conditions or under adiabatic conditions.

The isothermal conditions are preferably obtained by transferring the heat developed by the reaction of the syngas feed to a cooling medium. Any suitable cooling medium can be used but preferably pressurized water is used.

In some embodiment, additional hydrogen and carbon oxides can be prepared in the process and then reacted in the catalytic converter to increase the production of methanol. In an embodiment, additional hydrogen is produced via water electrolysis and carbon dioxide can also be obtained from a carbon dioxide recovery section.

In an embodiment, condensation of the raw methanol containing gas is carried out within the same pressure vessel of the converter, preferably after cooling in the feed effluent heat exchanger.

### Description of the figure

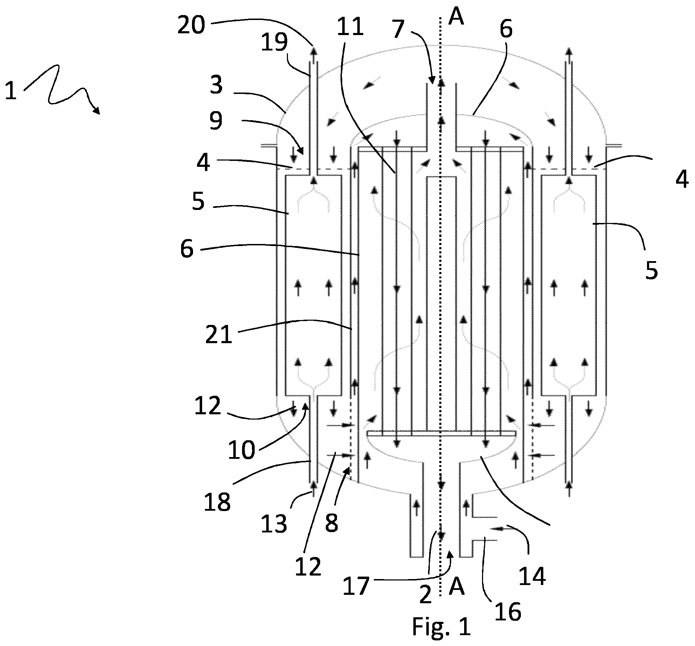

Fig. 1 is a schematic representation of a converter according to an embodiment of the invention.

### Detailed description of the preferred embodiments

The converter 1 shown in Fig. 1 comprises a pressure vessel 3 provided with a nozzle 16 for a syngas feed 14 and an outlet 17 for a raw methanol gas 2.

The pressure vessel further includes an annular catalytic bed 4, heat exchanger plates 5 and a feed-effluent heat exchanger 6.

The feed-effluent heat exchanger 6 is arranged centrally in the converter 1 along an axis of symmetry (A-A) of the pressure vessel 3. The catalytic bed 4 has an annular shape and is arranged concentrically around said feed-effluent heat exchanger 6 so to enclose the heat exchanger 6.

The exchanger plates 5 are immersed in the catalytic bed 4 and are in charge of removing the heat developed by the methanol synthesis reaction to keep the reaction temperature within a target temperature range.

Fig. 1 discloses a preferred embodiment of a single-bed, plate-cooled isothermal reactor.

The feed-effluent heat exchanger 6 comprises a first side 7 and a second side 8 wherein said first side 7 is in fluid communication with an inlet 9 of said catalytic bed 4 and said second side 8 is in fluid communication with an outlet 10 of said catalytic bed 4.

The feed-effluent heat exchanger 6 is a shell and tube heat exchanger provided with multiples tubes 11 arranged within a shell of the heat exchanger. The shelltube heat exchanger is arranged vertically within the converter so that the multiples tubes 11 are parallel to the axis of symmetry A-A of the reactor. The tubes 11 are in fluid communication with the outlet 10 of said catalytic bed 4 and with the outlet 17 of the pressure vessel.

In a variant (not shown), also the feed-effluent heat exchanger is a plate heat exchanger with heat exchange elements in the form of plates instead of tubes.

The outlet 10 of the catalytic bed 4 communicates with the tubes 11 of the heat exchanger 6 via a collector 21. The collector 21 is part of the shell of the feed-effluent heat exchanger 6 and extends along the entire length of said exchanger 6.

The shell side of the feed-effluent heat exchanger 6 is in fluid communication with a nozzle 16 of the pressure vessel 3 and with the inlet 9 of the catalytic bed 4.

An opening can be present on the pressure vessel 3 of the converter 1 to allow the extraction of the feed-effluent heat exchanger 6 from the pressure vessel 3 for maintenance and replacement purposes.

The heat exchanger plates 5 are also provided with a distribution system 18 arranged to supply a cooling medium 13, such as pressurized water, to the above-mentioned exchanger plates 5 and with a collector 19 arranged to collect the cooling medium 20 outside the converter 1.

The catalytic converter 1 works as follows: a syngas feed 14 enters the converter via nozzle 16 and crosses the shell side of the feed-effluent heat exchanger 6 wherein it absorbs heat from a hot methanol containing gas 12 which flows into the tubes 11 of the heat exchange 6. As a result of this heat transfer process, the syngas feed is pre-heated. The pre-heated synthesis gas is then transferred through the first side 7 toward the inlet 9 of the catalytic bed and in sequence into the catalytic bed 4 itself.

Herein the pre-heated synthesis gas reacts over the catalytic bed 4 to generate the hot methanol containing gas 12 which is then collected and conveyed to the second side 8 of the feed-effluent heat exchanger 6. Further the hot methanol containing gas 12, after crossing the collector 21, is fed to the tubes 11.

It can be noted that the hot methanol containing gas crosses the tubes 11 counter currently with the syngas feed 14 before being discharged outside the converter 1 via the outlet 17 nozzle as raw methanol gas 2. As above disclosed, heat is indirectly transferred from the hot methanol containing gas 12 to the syngas feed 14 in said shell and tube heat exchanger 6.

## Claims

1. Catalytic converter (1) for the synthesis of methanol (2) comprising a pressure vessel (3) which includes:
a catalytic bed (4) configured to contain a catalyst and having an annular configuration;
a set of cooling elements;
said catalytic bed (4) being configured as either an adiabatic catalytic bed, wherein said cooling elements are downstream the catalytic bed, or an isothermal catalytic bed, wherein said cooling elements are arranged to be immersed in the catalyst of the catalytic bed (4);
an effluent heat exchanger (6) arranged to transfer heat from a hot methanol-containing gas (12) effluent from the catalytic bed (4) to a cooling medium;
said effluent heat exchanger (6) being located in a central cavity of the annular catalytic bed (4), so that said catalytic bed (4) is arranged concentrically around said effluent heat exchanger (6);
wherein said effluent heat exchanger (6) is a feed-effluent heat exchanger arranged to transfer heat from said hot methanol-containing gas (12) effluent from the catalytic bed (4) to a syngas feed (14) to be reacted, so that said syngas feed (14) is preheated before entering said catalytic bed (4);
wherein said effluent heat exchanger (6) comprises: a first side (7) for the syngas feed (14), wherein an output of said first side (7) is in communication with an inlet (9) of said catalytic bed (4); a second side (8) for the hot methanol-containing gas (12), wherein an inlet of said second side (8) is in communication with an outlet (10) of said catalytic bed (4);
said catalytic converter (1) further comprising a methanol condenser contained in said pressure vessel (3), wherein said methanol condenser is arranged downstream of and in fluid communication with said effluent heat exchanger (6), so to receive a cooled methanol-containing gas effluent from said second side (8) of said effluent heat exchanger (6) and to produce a liquid methanol-containing product.

2. Catalytic converter (1) according to claim 1, wherein said effluent heat exchanger (6) is a shell and tube heat exchanger comprising tubes (11) housed within a cylindrical shell.

3. Catalytic converter (1) according to claim 1, wherein said effluent heat exchanger (6) is a plate type heat exchanger with heat exchange elements in the form of plates, said plates being internally traversed by the syngas feed.

4. Catalytic converter (1) according to any of the previous claims, wherein said effluent heat-exchanger (6) is part of a heat exchanger train including a plurality of heat exchanger units connected in series either within or outside said catalytic converter (1).

5. Catalytic converter (1) according to any of the previous claims, further comprising a liquid-gas separator arranged downstream of said effluent heat exchanger (6).

6. Converter according to claim 5, wherein said liquid-gas separator is arranged downstream of said condenser.

7. Catalytic converter (1) according to any of the previous claims, further comprising a start-up heater.

8. Converter according to any of the previous claims wherein: the converter is a single-bed converter, said catalytic bed (4) being the sole catalytic bed of the converter.

9. Converter according to any of the previous claims, wherein the converter is isothermal and said cooling elements immersed in the catalytic bed are in the form of heat exchange plates internally traversed by a cooling medium.

10. Process for the synthesis of methanol (2) wherein a syngas feed (14) comprising a mixture of carbon oxides and hydrogen is subjected to a heating step and subsequently reacted over a catalyst to produce a hot methanol-containing gas (12);
said hot methanol-containing gas (12) is then cooled and transferred to a condensation step and wherein said heating of said syngas feed (14) and said cooling of said hot methanol-containing gas (12) are carried out in an effluent heat exchanger (6) so that heat of said hot methanol-containing gas (12) is indirectly transferred to said syngas feed (14);
and wherein said reaction of said syngas feed (14) over said catalyst, said heating of the syngas feed (14), and said cooling and said condensation of said hot methanol-containing gas (12) are carried out in a catalytic converter (1) according to any of the claims 1 to 9 and
wherein said reaction between said syngas feed (14) over said catalyst is performed under adiabatic condition or isothermal condition.

11. Process according to claim 10, wherein said isothermal condition is obtained by transferring the heat developed by the reaction of said syngas feed (14) to a cooling medium (13).

12. Process according to claim 11, wherein said condensation of said hot methanol-containing gas (12) is carried out in sequence after said cooling in said catalytic converter (1).

13. Process according to any of claims 10 to 12, wherein said cooling medium (13) is pressurised water.

## Patentansprüche

1. Katalytischer Konverter (1) zur Synthese von Methanol (2), umfassend einen Druckbehälter (3), der Folgendes umfasst:
ein Katalysatorbett (4), das zur Aufnahme eines Katalysators eingerichtet ist und eine ringförmige Konfiguration aufweist;
einen Satz von Kühlelementen;
wobei das Katalysatorbett (4) entweder als adiabatisches Katalysatorbett ausgebildet ist, bei dem sich die Kühlelemente stromabwärts des Katalysatorbetts befinden, oder als isothermes Katalysatorbett, bei dem die Kühlelemente so angeordnet sind, dass sie in den Katalysator des Katalysatorbetts (4) eingetaucht sind;
einen Abgaswärmetauscher (6), der so angeordnet ist, dass er Wärme von einem heißen, methanolhaltigen Abgas (12) aus dem Katalysatorbett (4) an ein Kühlmedium überträgt;
wobei der Abgaswärmetauscher (6) in einem zentralen Hohlraum des ringförmigen Katalysatorbetts (4) angeordnet ist, sodass das Katalysatorbett (4) konzentrisch um den Abgaswärmetauscher (6) herum angeordnet ist;
wobei der Abgaswärmetauscher (6) ein Zufuhr-Abgas-Wärmetauscher ist, der so angeordnet ist, dass er Wärme von dem heißen, methanolhaltigen Abgas (12) aus dem Katalysatorbett (4) auf eine zur Reaktion bestimmte Synthesegas-Zufuhr (14) überträgt, sodass die Synthesegas-Zufuhr (14) vor ihrem Eintritt in das Katalysatorbett (4) vorgewärmt wird;
wobei der Abgaswärmetauscher (6) umfasst: eine erste Seite (7) für die Synthesegas-Zufuhr (14), wobei ein Auslass der ersten Seite (7) mit einem Einlass (9) des Katalysatorbetts (4) in Verbindung steht; eine zweite Seite (8) für das heiße, methanolhaltige Abgas (12), wobei ein Einlass der zweiten Seite (8) mit einem Auslass (10) des Katalysatorbetts (4) in Verbindung steht;
wobei der katalytische Konverter (1) ferner einen in dem Druckbehälter (3) enthaltenen Methanolkondensator umfasst, wobei der Methanolkondensator stromabwärts von und in Fluidverbindung mit dem Abgaswärmetauscher (6) angeordnet ist, um ein gekühltes, methanolhaltiges Abgas von der zweiten Seite (8) des Abgaswärmetauschers (6) aufzunehmen und ein flüssiges, methanolhaltiges Produkt zu erzeugen.

2. Katalytischer Konverter (1) nach Anspruch 1, wobei der Abgaswärmetauscher (6) ein Rohrbündelwärmetauscher ist, der Rohre (11) umfasst, die in einem zylindrischen Mantel untergebracht sind.

3. Katalytischer Konverter (1) nach Anspruch 1, wobei der Abgaswärmetauscher (6) ein Plattenwärmetauscher mit Wärmeaustauschelementen in Form von Platten ist, wobei die Platten im Inneren von der Synthesegas-Zufuhr (14) durchströmt werden.

4. Katalytischer Konverter (1) nach einem der vorstehenden Ansprüche, wobei der Abgaswärmetauscher (6) Teil einer Wärmetauscherkette ist, die eine Mehrzahl von Wärmetauscher-Einheiten umfasst, die entweder innerhalb oder außerhalb des Katalysators (1) in Reihe geschaltet sind.

5. Katalytischer Konverter (1) nach einem der vorstehenden Ansprüche, der ferner einen stromabwärts des Abgaswärmetauschers (6) angeordneten Flüssigkeits-Gas-Trenner umfasst.

6. Katalytischer Konverter (1) nach Anspruch 5, wobei der Flüssigkeits-Gas-Trenner stromabwärts des Methanolkondensators angeordnet ist.

7. Katalytischer Konverter (1) nach einem der vorstehenden Ansprüche, der ferner eine Anheizvorrichtung umfasst.

8. Katalytischer Konverter (1) nach einem der vorstehenden Ansprüche, wobei der Konverter ein Einbettkatalysator ist, wobei das Katalysatorbett (4) das einzige Katalysatorbett des Konverters ist.

9. Katalytischer Konverter (1) nach einem der vorstehenden Ansprüche, wobei der Konverter isotherm ist und die in das Katalysatorbett (4) eingetauchten Kühlelemente die Form von Wärmeaustauschplatten haben, die im Inneren von einem Kühlmedium durchströmt werden.

10. Verfahren zur Synthese von Methanol (2), bei dem eine Synthesegas-Zufuhr (14), die ein Gemisch aus Kohlenstoffoxiden und Wasserstoff umfasst, einem Erwärmungsschritt unterzogen und anschließend über einem Katalysator zur Erzeugung eines heißen, methanolhaltigen Gases (12) zur Reaktion gebracht wird; wobei das heiße, methanolhaltige Gas (12) anschließend gekühlt und einem Kondensationsschritt zugeführt wird, und wobei die Erwärmung der Synthesegas-Zufuhr (14) und die Kühlung des heißen methanolhaltigen Gases (12) in einem Abgaswärmetauscher (6) durchgeführt werden, so dass Wärme des heißen, methanolhaltigen Gases (12) indirekt auf die Synthesegas-Zufuhr (14) übertragen wird;
und wobei die Reaktion der Synthesegas-Zufuhr (14) über dem Katalysator, die Erwärmung der Synthesegas-Zufuhr (14) sowie die Kühlung und die Kondensation des heißen, methanolhaltigen Gases (12) in einem katalytischen Konverter (1) gemäß einem der Ansprüche 1 bis 9 durchgeführt werden, und
wobei die Reaktion der Synthesegas-Zufuhr (14) über dem Katalysator unter adiabatischen oder isothermen Bedingungen durchgeführt wird.

11. Verfahren nach Anspruch 10, wobei die isothermen Bedingungen dadurch erreicht werden, dass die bei der Reaktion der Synthesegas-Zufuhr (14) entstehende Wärme an ein Kühlmedium (13) übertragen wird.

12. Verfahren nach Anspruch 11, wobei die Kondensation des heißen, methanolhaltigen Gases (12) sequenziell nach der Kühlung in dem katalytischen Konverter (1) durchgeführt wird.

13. Verfahren nach einem der Ansprüche 10 bis 12, wobei das Kühlmedium (13) unter Druck stehendes Wasser ist.

## Revendications

1. Convertisseur catalytique (1) pour la synthèse de méthanol (2) comportant un récipient sous pression (3) qui inclut :
un lit catalytique (4) configuré pour contenir un catalyseur et présentant une configuration annulaire ;
un ensemble d'éléments de refroidissement ;
ledit lit catalytique (4) étant configuré soit comme un lit catalytique adiabatique, dans lequel lesdits éléments de refroidissement sont en aval du lit catalytique, soit comme un lit catalytique isotherme, dans lequel lesdits éléments de refroidissement sont agencés pour être immergés dans le catalyseur du lit catalytique (4) ;
un échangeur de chaleur d'effluent (6) agencé pour transférer de la chaleur depuis un effluent gazeux chaud (12) contenant du méthanol, provenant du lit catalytique (4), vers un milieu de refroidissement ;
ledit échangeur de chaleur d'effluent (6) étant situé dans une cavité centrale du lit catalytique annulaire (4), de sorte que ledit lit catalytique (4) est agencé de manière concentrique autour dudit échangeur de chaleur d'effluent (6) ;
dans lequel ledit échangeur de chaleur d'effluent (6) est un échangeur de chaleur alimentation-effluent agencé pour transférer de la chaleur depuis ledit effluent gazeux chaud (12) contenant du méthanol, provenant du lit catalytique (4), vers une alimentation en gaz de synthèse (14) destinée à réagir, de sorte que ladite alimentation en gaz de synthèse (14) est préchauffée avant d'entrer dans ledit lit catalytique (4) ;
dans lequel ledit échangeur de chaleur d'effluent (6) comporte : un premier côté (7) pour l'alimentation en gaz de synthèse (14), dans lequel une sortie dudit premier côté (7) est en communication avec une entrée (9) dudit lit catalytique (4) ; un deuxième côté (8) pour le gaz chaud (12) contenant du méthanol, dans lequel une entrée dudit deuxième côté (8) est en communication avec une sortie (10) dudit lit catalytique (4) ;
ledit convertisseur catalytique (1) comportant en outre un condenseur de méthanol contenu dans ledit récipient sous pression (3), dans lequel ledit condenseur de méthanol est agencé en aval et en communication fluidique avec ledit échangeur de chaleur d'effluent (6), de sorte à recevoir un effluent gazeux refroidi contenant du méthanol provenant dudit deuxième côté (8) dudit échangeur de chaleur d'effluent (6) et à produire un produit liquide contenant du méthanol.

2. Convertisseur catalytique (1) selon la revendication 1, dans lequel ledit échangeur de chaleur d'effluent (6) est un échangeur de chaleur à enveloppe et à tubes comportant des tubes (11) logés à l'intérieur d'une enveloppe cylindrique.

3. Convertisseur catalytique (1) selon la revendication 1, dans lequel ledit échangeur de chaleur d'effluent (6) est un échangeur de chaleur de type à plaques présentant des éléments d'échange de chaleur sous forme de plaques, lesdites plaques étant traversées intérieurement par l'alimentation en gaz de synthèse.

4. Convertisseur catalytique (1) selon l'une quelconque des revendications précédentes, dans lequel ledit échangeur de chaleur d'effluent (6) fait partie d'un train d'échangeurs de chaleur incluant une pluralité d'unités d'échangeurs de chaleur raccordées en série soit à l'intérieur, soit à l'extérieur dudit convertisseur catalytique (1).

5. Convertisseur catalytique (1) selon l'une quelconque des revendications précédentes, comportant en outre un séparateur liquide-gaz agencé en aval dudit échangeur de chaleur d'effluent (6).

6. Convertisseur selon la revendication 5, dans lequel ledit séparateur liquide-gaz est agencé en aval dudit condenseur.

7. Convertisseur catalytique (1) selon l'une quelconque des revendications précédentes, comportant en outre un réchauffeur de démarrage.

8. Convertisseur selon l'une quelconque des revendications précédentes dans lequel : le convertisseur est un convertisseur à lit unique, ledit lit catalytique (4) étant le seul lit catalytique du convertisseur.

9. Convertisseur selon l'une quelconque des revendications précédentes, dans lequel le convertisseur est isotherme et lesdits éléments de refroidissement immergés dans le lit catalytique sont sous forme de plaques d'échange de chaleur traversées intérieurement par un milieu de refroidissement.

10. Procédé de synthèse de méthanol (2), dans lequel une alimentation en gaz de synthèse (14) comportant un mélange d'oxydes de carbone et d'hydrogène est soumise à une étape de chauffage, puis mise à réagir sur un catalyseur pour produire un gaz chaud (12) contenant du méthanol ;
ledit gaz chaud (12) contenant du méthanol est ensuite refroidi et transféré vers une étape de condensation et dans lequel ledit chauffage de ladite alimentation en gaz de synthèse (14) et ledit refroidissement dudit gaz chaud (12) contenant du méthanol sont effectués dans un échangeur de chaleur d'effluent (6), de sorte que la chaleur dudit gaz chaud (12) contenant du méthanol est transférée indirectement à ladite alimentation en gaz de synthèse (14) ;
et dans lequel ladite réaction de ladite alimentation en gaz de synthèse (14) sur ledit catalyseur, ledit chauffage de l'alimentation en gaz de synthèse (14), et ledit refroidissement et ladite condensation dudit gaz chaud (12) contenant du méthanol sont effectués dans un convertisseur catalytique (1) selon l'une quelconque des revendications 1 à 9 et
dans lequel ladite réaction de ladite alimentation en gaz de synthèse (14) sur ledit catalyseur est effectuée en condition adiabatique ou en condition isotherme.

11. Procédé selon la revendication 10, dans lequel ladite condition isotherme est obtenue en transférant la chaleur dégagée par la réaction de ladite alimentation en gaz de synthèse (14) vers un milieu de refroidissement (13).

12. Procédé selon la revendication 11, dans lequel ladite condensation dudit gaz chaud (12) contenant du méthanol est effectuée en séquence après ledit refroidissement dans ledit convertisseur catalytique (1).

13. Procédé selon l'une quelconque des revendications 10 à 12, dans lequel ledit milieu de refroidissement (13) est de l'eau sous pression.
